# EUROPÄISCHE PATENTSCHRIFT

(11) **EP 3 573 943 B1**
(45) Veröffentlichungstag und Bekanntmachung des Hinweises auf die Patenterteilung: **17.03.2021**
(21) Anmeldenummer: 18700686.1
(22) Anmeldetag: 24.01.2018
(51) Int. Cl.: C07C 7/04, C07C 7/08, C07C 11/167, B01D 3/14

(54) **VERFAHREN ZUR GEWINNUNG VON REIN-1,3-BUTADIEN**
METHOD FOR OBTAINING PURE 1,3-BUTADIENE
PROCÉDÉ D'OBTENTION DE 1,3-BUTADIÈNE PUR

(30) Priorität: 25.01.2017 EP 17153120
(43) Veröffentlichungstag der Anmeldung: 04.12.2019
(73) Patentinhaber: BASF SE, 67056 Ludwigshafen am Rhein (DE)
(72) Erfinder: HEIDA, Bernd, 67158 Ellerstadt (DE); KELLER, Tobias, 67056 Ludwigshafen (DE); WEIDERT, Jan-Oliver, 67056 Ludwigshafen (DE)
(86) Internationale Anmeldenummer: PCT/EP2018/051613
(87) Internationale Veröffentlichungsnummer: WO 2018/138100

(56) Entgegenhaltungen:
- WO-A1-2005/075388
- WO-A1-2013/083536
- DE-A1- 2 251 051
- DE-A1- 10 105 660

## Beschreibung

Die vorliegende Erfindung betrifft ein Verfahren zur Gewinnung von Rein-1,3-Butadien aus einem Roh-C₄-Schnitt.

1,3-Butadien wird großtechnisch in der Regel aus so genannten C₄-Schnitten gewonnen, d.h. aus Gemischen von Kohlenwasserstoffen, worin die C₄-Kohlenwasserstoffe, insbesondere 1-Buten, i-Buten sowie 1,3-Butadien überwiegen.

C₄-Schnitte werden beispielsweise bei der Herstellung von Ethylen und Propylen durch thermisches Spalten, üblicherweise in Steamcrackern, insbesondere Naphtha- oder Gas-Crackern, erhalten. Weiterhin werden 1,3-Butadien enthaltende C₄-Schnitte bei der katalytischen Dehydrierung von n-Butan und/oder n-Buten erhalten. Als Ausgangsgasgemisch für die oxidative Dehydrierung von n-Butenen zu 1,3-Butadien kann jedes beliebige n-Butene enthaltende Gemisch benutzt werden. n-Butene enthaltende Gasgemische, die als Ausgangsgas in der oxidativen Dehydrierung von n-Butenen zu 1,3-Butadien eingesetzt werden, können durch nicht-oxidative Dehydrierung von n-Butan enthaltenden Gasgemischen hergestellt werden. Die 1,3-Butadien enthaltenden C₄-Schnitte werden nachfolgend als Roh-C₄-Schnitte bezeichnet. Sie enthalten neben geringen Mengen an C₃- und C₅-Kohlenwasserstoffen in der Regel Acetylene (Methylacetylen, Ethylacetylen und Vinylacetylen).

Es ist allgemein bekannt, dass Rein-1,3-Butadien aus Roh-C₄-Schnitten durch eine Abfolge bestimmter Verfahrensschritte gewonnen werden kann, bei denen aus dem Roh-C₄-Schnitt zunächst ein Roh-1,3-Butadien gewonnen und das Roh-1,3-Butadien dann weiter aufgereinigt wird, um daraus das Rein-1,3-Butadien zu gewinnen. Roh-1,3-Butadien ist ein Gemisch, das etwa 90 bis 99,5 Gew.-% 1,3-Butadien, insbesondere 98 bis 99 Gew.-% 1,3-Butadien, enthält. Die Spezifikationsanforderungen für Rein-1,3-Butadien sehen häufig einen Mindestgehalt an 1,3-Butadien von 99,6 Gew.-% und einen maximal zulässigen Gehalt an Acetylenen sowie an 1,2-Butadien von jeweils 20 ppm bezogen auf die Masse des Rein-1,3-Butadiens vor.

Die Gewinnung von 1,3-Butadien aus C₄-Schnitten ist wegen der geringen Unterschiede in den relativen Flüchtigkeiten der Komponenten eine komplexe Trennaufgabe. Daher führt man eine Extraktivdestillation durch, d.h. eine Destillation unter Zugabe eines selektiven Lösungsmittels, das einen höheren Siedepunkt als das aufzutrennende Gemisch aufweist und das die Unterschiede in den relativen Flüchtigkeiten der aufzutrennenden Komponenten erhöht. Das so erhaltene Roh-1,3-Butadien wird, um den Spezifikationsanforderungen zu entsprechen, destillativ zu Rein-1,3-Butadien aufgereinigt.

Beispielsweise wird gemäß der WO 2011/110562 A1 ein Roh-C₄-Schnitt selektiv hydriert, aus dem selektiv hydrierten C₄-Schnitt anschließend hochsiedende Bestandteile abgetrennt, der verbleibende C₄-Schnitt dann weiter durch Extraktivdestillation aufgearbeitet, um Roh-1,3-Butadien zu gewinnen. Das Roh-1,3-Butadien wird durch Reindestillation weiter zu Rein-1,3-Butadien aufgereinigt.

Die DE 101 05 660 offenbart ein Verfahren zur Gewinnung von Roh-1,3-Butadien durch Extraktivdestillation aus einem C₄-Schnitt mit einem selektiven Lösungsmittel. Das Verfahren wird in einer Trennwandkolonne (TK) durchgeführt, in der eine Trennwand (T) in Kolonnenlängsrichtung unter Ausbildung eines ersten Teilbereichs (A), eines zweiten Teilbereichs (B) und eines unteren gemeinsamen Kolonnenbereichs (C) angeordnet ist und der eine Extraktivwaschkolonne (K) vorgeschaltet ist.

Nach der WO 2013/083536 wird ein dampfförmiger gereinigter Roh-C₄-Schnitt als Einsatzstrom für eine Extraktivdestillation bereitgestellt, indem der flüssige Roh-C₄-Schnitt im oberen Drittel einer Destillationskolonne unter Ausbildung eines Verstärkungs- und eines Abtriebsteils zugeführt wird und aus der Destillationskolonne ein C₃-Kohlenwasserstoffe enthaltender Kopfstrom, ein C₄-Oligomere und -Polymere sowie die C₅₊-Kohlenwasserstoffe enthaltender Sumpfstrom und aus dem Abtriebsteil der dampfförmige gereinigte Roh-C₄-Schnitt als Seitenstrom abgezogen werden.

Den Verfahren zur Extraktivdestillation von C₄-Schnitten unter Verwendung von selektiven Lösungsmitteln ist gemeinsam, dass sich durch Gegenstromführung des aufzutrennenden C₄-Schnittes in Dampfform mit dem flüssigen selektiven Lösungsmittel bei geeigneten thermodynamischen Bedingungen, in der Regel bei niedrigen Temperaturen, meist im Bereich von 20 bis 80°C und bei moderaten Drücken, häufig bei etwa 3 bis etwa 6 bar, das selektive Lösungsmittel mit den Komponenten aus dem C₄-Schnitt belädt, zu denen es eine höhere Affinität hat, wogegen die Komponenten, zu denen das selektive Lösungsmittel eine geringere Affinität hat, in der Dampfphase verbleiben und als Kopfstrom abgezogen werden. Aus dem beladenen Lösungsmittelstrom werden anschließend unter geeigneten thermodynamischen Bedingungen, d.h. bei höherer Temperatur und/oder niedrigerem Druck die Komponenten fraktioniert aus dem selektiven Lösungsmittel freigesetzt.

1,3-Butadien ist eine polymerisationsfähige Verbindung und kann in verschiedenen Bereichen der Anlage unerwünschte polymere Ablagerungen bilden, die je nach Molekulargewicht und Vernetzungsgrad gummiartig oder spröde (so genannte Popcorn-Polymere) sein können. Die gummiartigen Beläge behindern den Wärmeübergang und führen zur Verringerung von Leitungsquerschnitten. Die Bildung von Popcorn-Polymeren kann schwere Schäden im Inneren der Anlage verursachen und zum Bersten von Kondensatoren und Leitungen führen. Die Ablagerungen müssen regelmäßig mit großem Aufwand und verlustreichen Stillstandzeiten aus Kolonnen und Rohrleitungen entfernt werden.

Als eine Hauptursache für die Bildung der Polymere wurde die Anwesenheit geringer Mengen an molekularem Sauerstoff im unteren ppm-Bereich oder darunter erkannt. Molekularer Sauerstoff kann z.B. über Butadienperoxid oder Hämatit als Radikalstarter die radikalische Polymerisation von 1,3-Butadien auslösen. Molekularer Sauerstoff gelangt durch kleinste Undichtigkeiten der Anlagenteile und über die in das Verfahren eingeleiteten Ströme in die Anlage.

Um den Sauerstoffgehalt unter einer vorbestimmten Grenzkonzentration zu halten, kann man aus den verschiedenen Kondensatoren, wo sich Sauerstoff, Stickstoff und andere Inertgase aufkonzentrieren, periodisch oder kontinuierlich einen Teil der Gasphase als Auslassstrom (Vent) aus dem Verfahren ausschleusen. Mit dem Auslassstrom entweicht neben molekularem Sauerstoff und Inertgasen, die darin nur in sehr niedriger Konzentration enthalten ist, eine beträchtliche Menge an Wertprodukt aus dem Verfahren. Es ist daher unerlässlich, den Sauerstoffgehalt in den relevanten Anlagenteilen durch Sauerstoffdetektoren zu überwachen und die Auslassströme auf das erforderliche Volumen zu begrenzen, um den Sauerstoffgehalt unter der Grenzkonzentration zu halten und gleichzeitig den C₄-Verlust auf ein Minimum zu beschränken. So lässt sich empirisch ein Gasvolumen ermitteln, das man gezielt aus der Anlage entweichen lässt. Beim kontrollierten Ausschleusen von Auslassströmen nach Maßgabe der Sauerstoff-Messung werden üblicherweise Sauerstoff-Restgehalte von 3-10 ppm im Auslassstrom vorgegeben. Nachteiligerweise sind die Sauerstoffdetektoren störanfällig und wartungsintensiv.

In den meisten Fällen wird bei den Extraktivdestillationsverfahren getrennt vom Roh-1,3-Butadien außerdem ein C₄-Acetylene enthaltendes Gas desorbiert. Da C₄-Acetylene chemisch instabil und explosiv sind, ist es übliche Praxis, das C₄-Acetylene enthaltende Gas aus Sicherheitsgründen mit einem anderen prozessinternen Strom so zu verdünnen, dass die Konzentration an C₄-Acetylene sicher unter der zerfallsgefährdeten Konzentration bleibt, z.B. bei 30 - 40 Vol% bei 1,7 bar absolut. Beispielsweise wird ein im Wesentlichen Raffinat 1 enthaltender Brüdenstrom der Extraktionskolonne als Verdünnungsgas verwendet. Das verdünnte C₄-Acetylene enthaltende Gas wird meistens dem Cracker Feedstock beigemischt, manchmal thermisch verwertet oder der Fackel zugeführt.

Der vorliegenden Erfindung liegt die Aufgabe zugrunde, ein effizientes Verfahren zur Gewinnung von Rein-1,3-Butadien aus einem Roh-C₄-Schnitt bereitzustellen, insbesondere ein Verfahren, bei dem der Produktionsbetrieb lange aufrechterhalten werden kann und man dennoch eine hohe Ausbeute an Rein-1,3-Butadien erzielt.

Die Aufgabe wird gelöst durch ein Verfahren zur Gewinnung von Rein-1,3-Butadien aus einem Roh-C₄-Schnitt durch Extraktivdestillation mit einem selektiven Lösungsmittel, wobei man
a) einen flüssigen Roh-C₄-Schnitt in den Zulaufbereich einer Vordestillationskolonne einführt, welche in einem mittleren Abschnitt durch eine im Wesentlichen in Längsrichtung der Vordestillationskolonne ausgerichtete Trennwand in einen Zulaufbereich und einen Seitenabzugsbereich unterteilt ist, eine C₃-Kohlenwasserstoffe enthaltende Leichtsiederfraktion als Kopfstrom, einen gasförmigen C₄-Schnitt als Seitenstrom aus dem Seitenabzugsbereich und eine erste Hochsiederfraktion als Sumpfstrom abzieht,
b) den gasförmigen C₄-Schnitt in wenigstens einer Extraktionskolonne mit einem selektiven Lösungsmittel in Kontakt bringt, wobei man eine Butane und Butene enthaltende Kopffraktion und eine 1,3-Butadien und selektives Lösungsmittel enthaltende Sumpffraktion erhält,
c) aus der Sumpffraktion in wenigstens einer Ausgaserkolonne Roh-1,3-Butadien desorbiert, wobei man ein ausgegastes selektives Lösungsmittel erhält und das ausgegaste selektive Lösungsmittel in die Extraktionskolonne zurückführt, und
d) wenigstens einen Teil des Roh-1,3-Butadiens einer Reindestillationskolonne zuführt und eine zweite Hochsiederfraktion abtrennt und einen gasförmigen Auslassstrom abzieht und die zweite Hochsiederfraktion in einen unteren Abschnitt der Vordestillationskolonne zurückführt.

Aus Investitionskostengründen kann es vorteilhaft sein, anstelle einer Extraktionskolonne zwei Kolonnen so zu koppeln, dass sie thermodynamisch einer Kolonne der doppelten Trennstufenzahl entsprechen. Die Extraktions- und die Ausgaserkolonne können auch je ganz oder teilweise in einer integrierten Extraktions- und Ausgaserkolonne integriert sein. Der Extraktions- und Ausgaserkolonne können (eine) weitere Extraktionskolonne(n) vorgeschaltet und/oder (eine) weitere Ausgaserkolonne(n) nachgeschaltet sein. Wenn der integrierten Extraktions- und Ausgaserkolonne eine weitere Ausgaserkolonne nachgeschaltet ist, wird im vorliegenden Dokument bei der integrierten Kolonne von einer Extraktions- und Vorausgaserkolonne gesprochen.

In den bevorzugten Ausführungsformen des erfindungsgemäßen Verfahrens werden gasförmige Auslassströme aus den Kolonnen, die erforderlich sind um die Konzentration an molekularem Sauerstoff unterhalb einer vorbestimmten Grenzkonzentration zu halten, auf Auslässe konsolidiert, die zur Ausschleusung anderer Komponenten im Verfahren ohnehin vorgesehen sind. Da es sich hierbei im Vergleich zu gängigen Auslassströmen um volumenmäßig große Ströme handelt, gelingt es den Sauerstoffgehalt statt üblicher, überwachter 3-10 ppm sicher unter der Nachweisgrenze industrieüblicher Sauerstoffdetektoren zu halten.

Vorzugsweise führt man den Auslassstrom aus der Reindestillationskolonne zusammen mit dem Brüden der Vordestillationskolonne durch den Kopfkondensator der Vordestillationskolonne. Dies gestattet den Volumenstrom des Auslassstroms aus der Reindestillationskolonne hochzufahren, um als Auslass für Sauerstoffspuren in der Reindestillationskolonne zu dienen und den Sauerstoffgehalt in der Reindestillationskolonne unter die Nachweisgrenze üblicher Sauerstoffdetektoren zu drücken. Im Auslassstrom aus der Reindestillationskolonne enthaltenes 1,3-Butadien kondensiert im Kopfkondensator der Vordestillationskolonne und wird im Verfahren zurückgehalten.

Ein dezidierter Auslassstrom aus der Ausgaserkolonne, in welcher Roh-1,3-Butadien desorbiert wird, wird entbehrlich, wenn das Roh-1,3-Butadien gasförmig aus der Ausgaserkolonne abgezogen und gasförmig der Reindestillationskolonne zugeführt wird. Hierzu kondensiert man das desorbierte Roh-1,3-Butadien teilweise, führt den kondensierten Teil des Roh-1,3-Butadiens als Rücklauf in die Ausgaserkolonne und/oder in eine nachstehend beschriebene Nachwaschzone und führt den anderen Teil des Roh-1,3-Butadiens gasförmig der Reindestillationskolonne zu. Der Volumenstrom des gasförmig abgezogenen Roh-1,3-Butadiens beträgt ein Vielfaches des Volumenstroms eines üblichen Auslassstroms für Sauerstoff; so kann der Sauerstoffgehalt in der Ausgaserkolonne unter die Nachweisgrenze üblicher Sauerstoffdetektoren gedrückt werden.

Weiterhin wird vorgeschlagen, als Verdünnungsgas für C₄-Acetylene einen Strom zu verwenden, der gleichzeitig als Auslassstrom für Sauerstoff und Inertgase aus der Extraktionskolonne dient. Man verdünnt das C₄-Acetylene enthaltende Gas vorzugsweise mit nicht kondensierten Bestandteilen des Brüdens der Extraktionszone, d.h. der Auslass ist nicht im Brüdenraum der Extraktionskolonne sondern am oder hinter dem Kondensator der Extraktionskolonne angeordnet, so dass dieser gleichzeitig als überdimensionierter Auslassstrom dient. C4 Verluste über den Auslassstrom treten somit nicht mehr auf.
Der Roh-C₄-Schnitt wird in der Regel flüssig in die Vordestillationskolonne eingeführt. Eine C₃-Kohlenwasserstoffe enthaltende Leichtsiederfraktion wird als Kopfstrom abgezogen. Ein gasförmiger C₄-Schnitt wird aus der Vordestillationskolonne als Seitenstrom abgezogen. Eine erste Hochsiederfraktion wird aus der Vordestillationskolonne als Sumpfstrom abgezogen. Die Vordestillationskolonne bewirkt gleichzeitig eine Vorreinigung und Verdampfung des Roh-C₄-Schnitts. Ein separater Verdampfer für den gereinigten Roh-C₄-Schnitt entfällt. Die Vordestillationskolonne kann z.B. bei einer Sumpftemperatur von 50 bis 80.°C und bei einem Druck von 4 bis 8 bar betrieben werden. All die im vorliegenden Dokument angegebenen Drücke sind Absolutdrücke.

Über die erste Hochsiederfraktion kann der wesentliche Anteil der im Roh-C₄-Schnitt enthaltenden C₅₊-Kohlenwasserstoffe und der im Roh-C₄-Schnitt enthaltenen C₄-Oligomere und -Polymere ausgeschleust werden. Da weniger C₅₊-Komponenten, insbesondere polymerisierbare C₅-Diene wie Isopren oder cis-2-Pentadien aus der Vordestillation der Extraktivdestillation zugeführt werden, reichern sich diese im selektiven Lösungsmittel weniger stark an. Über die erste Hochsiederfraktion werden auch Karbonyle wie Formaldehyd, Acetaldehyd, Propionaldehyd, n-Butyraldehyd, Crotonaldehyd, Aceton, Methylethylketon oder Acrolein, ausgeschleust.

Der flüssige Roh-C₄-Schnitt wird der Vordestillationskolonne zweckmäßigerweise seitlich unter Ausbildung eines oberhalb des Zulaufs gelegenen Verstärkungsteils und eines unterhalb des Zulaufs gelegenen Abtriebsteils zugeführt. Die Vordestillationskolonne ist bevorzugt eine Bodenkolonne. Die Bodenkolonne weist insbesondere 30 bis 100 praktische Böden oder besonders bevorzugt 50 bis 70 praktische Böden auf. Der seitliche Zulauf teilt die Zahl der darüberliegenden Böden und die Zahl der darunterliegenden Böden vorzugsweise im Verhältnis 20:80 bis 80:20.

Die Vordestillationskolonne ist in einem mittleren Abschnitt durch eine im Wesentlichen in Längsrichtung der Vordestillationskolonnne ausgerichtete Trennwand in einen Zulaufbereich und einen Seitenabzugsbereich unterteilt. Dabei führt man den flüssigen Roh-C₄-Schnitt in den Zulaufbereich ein und zieht den gasförmigen C₄-Schnitt aus dem Seitenabzugsbereich ab. Die Komponenten des Roh-C₄-Schnitts können nicht direkt zum Seitenabzug und in den Seitenstrom gelangen, da sie beim Umströmen der Unter- bzw. Oberkante der Trennwand von herablaufendem Kondensat bzw. aufsteigendem Brüden aufgetrennt werden. Die Trennwand erstreckt sich im Allgemeinen über 4 bis 40 Böden, vorzugsweise über 6 bis 30 Böden.

In einer alternativen Ausführungsform kann die Vordestillationskolonne auch als eine Destillationskolonne ohne Trennwand ausgestaltet sein. Der Abzug des gasförmigen C₄-Schnitts erfolgt dann unterhalb des Zulaufs des Roh-C₄-Schnitts. Dies gewährleistet, dass die Komponenten des Roh-C₄-Schnitts nicht direkt zum Seitenabzug und in den Seitenstrom gelangen.

Den so gewonnenen gasförmigen gereinigten C₄-Schnitt bringt man in wenigstens einer Extraktionskolonne mit einem selektiven Lösungsmittel in Kontakt, wobei man eine Butane und Butene enthaltende Kopffraktion und eine 1,3-Butadien, C₄-Acetylene und selektives Lösungsmittel enthaltende Sumpffraktion erhält. Normalerweise bringt man den gasförmigen C₄-Schnitt mit dem selektiven Lösungsmittel in Kontakt, indem man den gasförmigen C₄-Schnitt in wenigstens einem Abschnitt der Extraktionskolonne(n) im Gegenstrom zum selektiven Lösungsmittel führt.

Druck und Temperatur stellt man in der Extraktionskolonne so ein, dass diejenigen Komponenten des C₄-Schnittes, für die das selektive Lösungsmittel eine geringere Affinität als für 1,3-Butadien hat, insbesondere die Butane und die Butene, im Wesentlichen in der Gasphase verbleiben, wogegen 1,3-Butadien sowie Acetylene und weitere Kohlenwasserstoffe, für die das selektive Lösungsmittel eine höhere Affinität als für 1,3-Butadien hat, vom selektiven Lösungsmittel im Wesentlichen vollständig absorbiert werden. Die Extraktionskolonne kann z.B. bei einer Temperatur von 20 bis 80°C und bei einem Druck von 3 bis 6 bar betrieben werden. So erhält man die Butane und Butene enthaltende Kopffraktion und die 1,3-Butadien, C₄-Acetylene und selektives Lösungsmittel enthaltende Sumpffraktion. Butane und Butene werden über Kopf abgezogen. Die Kopffraktion wird üblicherweise als Raffinat 1 bezeichnet.

Die Sumpffraktion enthält neben dem Lösungsmittel und 1,3-Butadien in der Regel weitere Kohlenwasserstoffe, für die das selektive Lösungsmittel eine höhere Affinität als für 1,3-Butadien hat, wie z.B. C₄-Acetylene. Normalerweise führt man deshalb eine fraktionierte Desorption durch, wobei die im selektiven Lösungsmittel absorbierten Kohlenwasserstoffe in der umgekehrten Reihenfolge ihrer Affinität zum selektiven Lösungsmittel desorbiert werden. Demgemäß desorbiert man aus der Sumpffraktion neben Roh-1,3-Butadien vorzugsweise außerdem ein C₄-Acetylene, insbesondere Vinylacetylen, enthaltendes Gas als separate Fraktion.

In einer Ausführungsform leitet man die Sumpffraktion in eine Extraktions- und Vorausgaserkolonne. Der obere Teil der Extraktions- und Vorausgaserkolonne wirkt als Abtriebsteil, in dem die im Lösungsmittel noch gelösten Butane und Butene sowie andere Leichtsieder ausgetrieben und über Kopf abgezogen werden können. Das Kopfprodukt der Extraktions- und Vorausgaserkolonne kann wieder der Extraktionskolonne zugeführt werden. Roh-1,3-Butadien, das neben 1,3-Butadien geringe Mengen Methylacetylen, 1,2-Butadien und C₅₊-Kohlenwasserstoffe enthält, kann als Seitenabzug aus der Extraktions- und Vorausgaserkolonne abgezogen werden. Vorentgastes Lösungsmittel, das noch verschiedene C₄-Komponenten, wie Vinylacetylen, enthält, fällt im Sumpf der Extraktions- und Vorausgaserkolonne an. Die Extraktions- und Vorausgaserkolonne kann z.B. bei einer Sumpftemperatur von 20 bis 80°C und bei einem Druck von 3 bis 6 bar betrieben werden.

Vorzugsweise leitet man das im Sumpf der Extraktions- und Vorausgaserkolonne anfallende vorentgaste Lösungsmittel in eine Ausgaserkolonne, in der man weitere Kohlenwasserstoffe, für die das selektive Lösungsmittel eine höhere Affinität als für 1,3-Butadien hat, z.B. C₄-Acetylene, desorbiert. Die Ausgaserkolonne kann z.B. bei einer Temperatur von 120 bis 200°C und bei einem Druck von 1,2 bis 6 bar betrieben werden.

Vorzugsweise zieht man das C₄-Acetylene enthaltende Gas als Seitenabzug aus der Ausgaserkolonne ab. Beispielsweise kann man das als Seitenabzug aus der Ausgaserkolonne abgezogene, C₄-Acetylene enthaltende Gas in einem Acetylenwäscher mit Wasser waschen, um selektives Lösungsmittel zurückzugewinnen. Der Acetylenwäscher kann als Seitenkolonne der Ausgaserkolonne ausgebildet sein. Das Waschwasser kann in den Lösungsmittelkreislauf recycliert werden, z.B. in die Ausgaserkolonne und/oder die Extraktions- und Vorausgaserkolonne. Wasserdampf, der vom gewaschenen C₄-Acetylene enthaltenden Gas mitgeführt wird, kann auskondensiert und ganz oder teilweise in den Acetylenwäscher zurückgeführt werden.

Um denjenigen Teil des 1,3-Butadiens rückzugewinnen, der erst in der Ausgaserkolonne desorbiert wird, kann man das Kopfprodukt der Ausgaserkolonne komprimieren und in die Extraktions- und Vorausgaserkolonne zurückführen. Geeigneterweise wird das Kopfprodukt der Ausgaserkolonne vor dem Komprimieren gekühlt, z.B. mittels eines Direktkühlers.

Am Sumpf der Ausgaserkolonne erhält man ein ausgegastes selektives Lösungsmittel, das zunächst zur Wärmerückgewinnung eingesetzt werden kann und nach einer Schlusskühlung in die Extraktionskolonne und gegebenenfalls in eine nachstehend beschriebene Nachwaschzone zurückgeführt werden kann.

Im Allgemeinen behandelt man das Roh-1,3-Butadien in einer Nachwaschzone mit ausgegastem selektiven Lösungsmittel. Dies hat den Vorteil, dass die im Roh-1,3-Butadien noch enthaltenen C₄-Acetylene ausgewaschen werden, bevor das Roh-1,3-Butadien in die Reindestillationskolonne gelangt. Das aus der Nachwaschzone ablaufende Lösungsmittel kann in die Extraktions- und Vorausgaserkolonne geleitet werden. Die Nachwaschzone kann von einer separaten Kolonne, z.B. einer der Extraktions- und Vorausgaserkolonne beigeordneten Seitenkolonne, gebildet sein. In einer geeigneten Ausführungsform ist die Nachwaschzone ein durch eine im Wesentlichen in Längsrichtung der Kolonne verlaufende Trennwand abgetrennter oberer Abschnitt der Extraktions- und Vorausgaserkolonne. In einem oberen Bereich der Extraktions- und Vorausgaserkolonne ist dann eine Trennwand in Kolonnenlängsrichtung angeordnet, unter Ausbildung eines der Extraktion dienenden ersten oberen Abschnitts, eines die Nachwaschzone bildenden zweiten oberen Abschnitts und eines der Desorption dienenden, sich unten an die Trennwand anschließenden dritten Abschnitts. Vorzugsweise ist die Trennwand außermittig angeordnet, und zwar dergestalt, dass die Querschnittsfläche der Nachwaschzone kleiner ist gegenüber der Querschnittsfläche der Extraktionszone.

Wenigstens einen Teil des Roh-1,3-Butadiens führt man einer Reindestillationskolonne zu. In der Reindestillationskolonne gewinnt man Rein-1,3-Butadien unter Abtrennung einer zweiten Hochsiederfraktion. Die zweite Hochsiederfraktion zieht man aus dem Sumpf der Reindestillationskolonne ab. Außerdem wird aus der Reindestillationskolonne ein Auslassstrom abgezogen. Der Auslassstrom aus der Reindestillationskolonne besteht im Wesentlichen aus 1,3-Butadien mit Spuren von Wasserdampf, Sauerstoff und Inertgasen. Der Auslassstrom aus der Reindestillationskolonne dient zum Ausschleusen von Sauerstoff und Inertgasen.

Die Reindestillationskolonne kann z.B. bei einer Sumpftemperatur von 40 bis 80°C und bei einem Druck von 2 bis 8 bar betrieben werden.

Der Auslassstrom aus der Reindestillationskolonne kann am Kopf der Reindestillationskolonne abgezogen werden und Rein-1,3-Butadien kann als Seitenstrom aus der Reindestillationskolonne abgezogen werden. Auf diese Weise kann Rein-1,3-Butadien mit einem Wassergehalt gewonnen werden, der niedriger ist als die physikalische Löslichkeit von Wasser in 1,3-Butadien.

In einer zweckmäßigen Ausführungsform werden der Auslassstrom und Rein-1,3-Butadien gemeinsam als Kopfdampf von der Reindestillationskolonne abgezogen und Rein-1,3-Butadien und Wasser werden aus dem Kopfdampf auskondensiert. Die nicht kondensierten Bestandteile werden als Auslassstrom aus der Reindestillationskolonne ausgeschleust oder wie nachstehend beschrieben zurückgeführt. Ein Teilstrom des kondensierten Rein-1,3-Butadiens wird nach Phasentrennung als Rücklauf in die Reindestillationskolonne gegeben, der andere Teil als Rein-1,3-Butadien abgezogen. Der Wassergehalt des so erhaltenen Rein-1,3-Butadiens entspricht der physikalischen Löslichkeit von Wasser in 1,3-Butadien.

Gemäß einer bevorzugten Ausführungsform des erfindungsgemäßen Verfahrens führt man den Auslassstrom aus der Reindestillationskolonne zusammen mit dem Brüden der Vordestillationskolonne durch den Kopfkondensator der Vordestillationskolonne. Im Allgemeinen wird die Reindestillationskolonne bei etwas niedrigerem Druck betrieben, als die Vordestillationskolonne. Man fördert die den Auslassstrom aus der Reindestillationskolonne dann aktiv, z.B. mit Hilfe eines Gebläses oder eines Kompressors, um ihn gegen das Druckgefälle zu fördern. Der Auslassstrom aus der Reindestillationskolonne kann z.B. am Kopf der Vordestillationskolonne oder in die Zufuhrleitung zum Kondensator eingeführt werden. Diese Ausführungsform gestattet, dass der Volumenstrom des Auslassstroms aus der Reindestillationskolonne in weiten Grenzen hochgefahren werden kann, um als Auslassstrom für Sauerstoffspuren in der Reindestillationskolonne zu dienen und den Sauerstoffgehalt in der Reindestillationskolonne unter die Nachweisgrenze üblicher Sauerstoffdetektoren zu drücken. Letztlich dient der Auslassstrom auch als Auslass für Sauerstoff. Dies verringert die Polymerisationsneigung des in der Reindestillationskolonne befindlichen 1,3-Butadiens, wodurch wiederum der Produktionsbetrieb länger aufrechterhalten werden kann. Im Auslassstrom aus der Reindestillationskolonne enthaltenes 1,3-Butadien geht bei dieser Verfahrensführung nicht verloren. Der Kopfkondensator der Vordestillationskolonne wird so betrieben, dass C₃-Kohlenwasserstoffe als Kopfstrom abgetrennt werden, höhere Kohlenwasserstoffe dagegen kondensiert und als Rücklauf auf die Vordestillationskolonne gegeben werden. Das 1,3-Butadien kondensiert daher im Kopfkondensator der Vordestillationskolonne per Massenbilanz vollständig, strömt in die Vordestillationskolonne und bildet einen Teil des daraus als Seitenstrom abgezogenen gasförmigen C₄-Schnitts.

Im erfindungsgemäßen Verfahrens führt man die zweite Hochsiederfraktion in einen unteren Abschnitt der Vordestillationskolonne zurück. In der zweiten Hochsiederfraktion gegebenenfalls enthaltenes 1,3-Butadien geht bei dieser Verfahrensführung nicht verloren. Dies schafft einen weiteren Freiheitsgrad im Betrieb der Reindestillationskolonne, z.B. die Möglichkeit, auf eine wechselnde Zusammensetzung des Roh-C₄-Schnitts flexibel zu reagieren und den Energieverbrauch der Anlage zu minimieren. Es ist möglich, den Sumpf der Reindestillationskolonne flexibler zu beheizen. Wird beispielsweise festgestellt, dass der Gehalt an hochsiedenden Verunreinigungen im anfallenden Rein-1,3-butadien steigt, kann man die Wärmezufuhr am Sumpf der Reindestillationskolonne verringern. Dies hat zur Folge, dass die Hochsieder in der Reindestillationskolonne weniger stark aufgetrieben werden. Jedoch wird 1,3-Butadien dann stärker abgetrieben. Dadurch steigt im Sumpf der Reindestillationskolonne der Gehalt an Rein-1,3-butadien. Jedoch geht dieses nicht verloren, da die sich im Sumpf der Kolonne sammelnde zweite Hochsiederfraktion nicht verworfen, sondern gemäß dieser bevorzugten Ausführungsform in den unteren Abschnitt der Vordestillationskolonne zurückgeführt wird. Man vermeidet dadurch also Verluste an Rein-1,3-Butadien, die bei einer Verringerung der Wärmezufuhr am Sumpf der Reindestillationskolonne sonst typischerweise auftreten. Dies bietet unter realen Betriebsbedingungen, die gelegentlich ein Nachjustieren der Sumpfbeheizung erforderlich machen, den zusätzlichen Vorteil einer weiteren Steigerung der Ausbeute an Rein-1,3-Butadien, bezogen auf das im Roh-C₄-Schnitt enthaltene 1,3-Butadien, ohne die Reinheit des Rein-1,3-Butadiens zu beeinträchtigen. Das Verfahren wird dadurch effizienter und wegen des zusätzlichen Freiheitsgrades einfacher und flexibler zu betreiben.

Gemäß einer weiteren bevorzugten Ausführungsform zieht man das Roh-1,3-Butadien, das man der Reindestillationskolonne zuführt, gasförmig von der Extraktions- und Vorausgaserkolonne ab und führt es gasförmig in die Reindestillationskolonne ein. Ist eine Nachwaschzone vorgesehen, gibt es einen durchgängigen Gaspfad von der Extraktions- und Vorausgaserkolonne über die Nachwaschzone zur Reindestillationskolonne. In einem Teilkondensator kondensiert man das Roh-1,3-Butadien teilweise und führt den kondensierten Teil des Roh-1,3-Butadiens als Rücklauf in die Kolonne bzw. in die Nachwaschzone. Der nicht kondensierte Teil des Roh-1,3-Butadiens wird gasförmig der Reindestillationskolonne zugeführt. Ein gasförmiger Ausleitstrom aus der Extraktions- und Vorausgaserkolonne ist nun nicht mehr erforderlich, da molekularer Sauerstoff und Inertgase mit dem gasförmigen Roh-1,3-Butadien abgezogen werden. Der Volumenstrom des gasförmig abgezogenen Roh-1,3-Butadiens beträgt ein Vielfaches des Volumenstroms eines üblichen Auslassstroms für Sauerstoff und Inertgase; so kann der Sauerstoffgehalt in der Extraktions- und Vorausgaserkolonne unter die Nachweisgrenze üblicher Sauerstoffdetektoren gedrückt werden. Als Auslass für den über das gasförmige Roh-1,3-butadien in die Reindestillationskolonne eingetragenen molekularen Sauerstoff dient der Auslassstrom aus der Reindestillationskolonne. Diese Ausführungsform ist also insbesondere dann bevorzugt, wenn man den Auslassstrom aus der Reindestillationskolonne zusammen mit dem Brüden der Vordestillationskolonne durch den Kopfkondensator der Vordestillationskolonne führt. Denn dann lässt sich ein zusätzlicher Eintrag molekularen Sauerstoffs in die Reindestillationskolonne besonders wirtschaftlich dadurch kompensieren, dass man die Menge des am Kopf der Reindestillationskolonne abgezogenen Auslassstroms erhöht und darüber somit mehr molekularen Sauerstoff abzieht.

Gemäß einer weiteren bevorzugten Ausführungsform sieht man einen gasförmigen Auslassstrom aus der Extraktionskolonne vor. Vorzugsweise ist der Auslass am oder hinter dem Kondensator, z.B. am Destillatsammler, angeordnet, d.h. man leitet die verbleibenden Gasbestandteile aus, die im Kopfkondensator der Extraktionskolonne nicht kondensiert werden. Diese nicht kondensierten Bestandteile bestehen im Wesentlichen aus Butanen, Butenen, Inertgasen wie Stickstoff und untergeordneten Mengen an molekularem Sauerstoff. Mit Vorteil kann man den Auslassstrom aus der Extraktionskolonne verwenden, um das C₄-Acetylene enthaltende Gas zu verdünnen, das in der Ausgaserkolonne desorbiert wird.

Der Roh-C₄-Schnitt enthält zumindest 1,3-Butadien, Butane, Butene und C₄-Acetylene. In vielen Fällen enthält der Roh-C₄-Schnitt 1,3-Butadien, Butane, Butene und C₄-Acetylene, C₃-Kohlenwasserstoffe und C₅₊-Kohlenwasserstoffe.

Bei dem Roh-C₄-Schnitt handelt es sich z.B. um einen Roh-C₄-Schnitt aus einem Naphtha Cracker.

Ein typischer Roh-C₄-Schnitt aus einem Naphtha Cracker weist die folgende Zusammensetzung in Gewichtsprozenten auf:

| | |
|---|---|
| Propan | 0-0,5 |
| Propen | 0-0,5 |
| Propadien | 0-0,5 |
| Propin | 0-0,5 |
| n-Butan | 3-10 |
| i-Butan | 1 -3 |
| 1-Buten | 10-20 |
| i-Buten | 10-30 |
| trans-2-Buten | 2-8 |
| cis-2-Buten | 2-6 |
| 1,3-Butadien | 15-85 |
| 1,2-Butadien | 0,1 - 1 |
| Ethylacetylen | 0,1-2 |
| Vinylacetylen | 0,1 -3 |
| C₅-Kohlenwasserstoffe | 0-0,5 |

Roh-C₄-Schnitte aus Naphtha-Crackern enthalten somit überwiegend Butane, Butene und 1,3-Butadien. Darüber hinaus sind geringe Mengen an sonstigen Kohlenwasserstoffen enthalten. C₄-Acetylene sind häufig bis zu einem Anteil von 5 Gew.-% oder auch bis zu 2 Gew.-% enthalten.

Als selektive Lösungsmittel kommen generell Substanzen oder Gemische in Frage, die einen höheren Siedepunkt als das aufzutrennende Gemisch sowie eine größere Affinität zu konjugierten Doppelbindungen und Dreifachbindungen als zu einfachen Doppelbindungen sowie Einfachbindungen aufweisen, bevorzugt dipolare, besonders bevorzugt dipolar-aprotische Lösungsmittel. Aus apparatetechnischen Gründen werden wenig oder nicht korrosive Substanzen bevorzugt. Geeignete selektive Lösungsmittel für das erfindungsgemäße Verfahren sind zum Beispiel Nitrile wie Acetonitril, Propionitril, Methoxypropionitril, Ketone wie Aceton, Furfurol, N-alkylsubstituierte niedere aliphatische Saureamide, wie Dimethylformamid, Diethylformamid, Dimethylacetamid, Diethylacetamid, N- Formylmorpholin, N-alkylsubstituierte cydische Saureamide (Lactame) wie N-Alkylpyrrolidone, insbesondere N-Methylpyrrolidon (NMP). Im Allgemeinen werden N- alkylsubstituierte niedere aliphatische Saureamide oder N-alkylsubstituierte cyclische Saureamide verwendet. Besonders vorteilhaft sind Dimethylformamid, Acetonitril, Furfurol und insbesondere N-Methylpyrrolidon.

Es können jedoch auch Mischungen dieser Lösungsmittel untereinander, zum Beispiel von N-Methylpyrrolidon mit Acetonitril, Mischungen dieser Lösungsmittel mit Colösungsmitteln wie Wasser, Alkoholen, insbesondere solchen mit 5 oder weniger Kohlenstoffatomen, wie Methylalkohol, Ethylalcohol, n-Propylalkohol, Isopropylalkohol, n-Butylalkohol, Isobutylalkohol, sec-Butylalkohol, tert-Butylalkohol, n-Pentylalkohol, oder alicyclische Alkohole wie Cyclopentanol, Diolen, wie Ethylenglycol, und/oder tert.-Butylether, zum Beispiel Methyl-tert-butylether, Ethyl-tert.-butylether, Propyl-tert.-butylether, n- oder iso-Butyl-tert- butylether eingesetzt werden.

Gemäß einer bevorzugten Ausführungsform des erfindungsgemäßen Verfahrens enthält das selektive Lösungsmittel wenigstens 80 Gew.-% N-Methylpyrrolidon. Bevorzugt enthält das selektive Lösungsmittel 85 bis 95 Gew.-% NMP und 5 bis 15 Gew.-% Wasser. Besonders geeignet ist N-Methylpyrrolidon, bevorzugt in wässriger Lösung, insbesondere mit 7 bis 9 Gew.-% Wasser, besonders bevorzugt mit 8,3 Gew.-% Wasser.

Darüber hinaus kann das selektive Lösungsmittel insbesondere noch Hilfsstoffe, Inhibitoren, Antischaummittel, organische Nebenkomponenten als Verunreinigung enthalten.

Die Erfindung wird durch die beigefügte Zeichnung und die nachfolgenden Beispiele näher veranschaulicht.

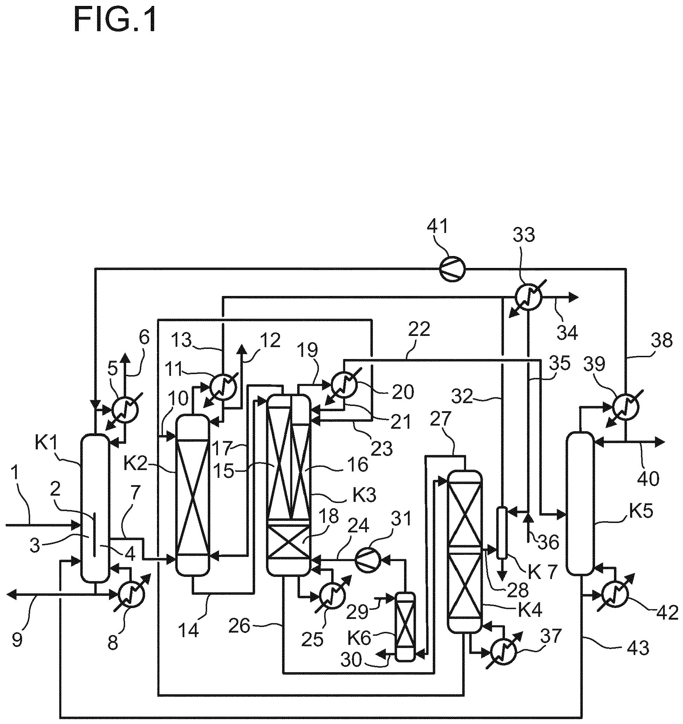

Figur 1 zeigt schematisch eine bevorzugte Anlage zur Durchführung des erfindungsgemäßen Verfahrens.

Ein flüssiger Roh-C₄-Schnitt, Strom 1, wird in die Vordestillationskolonne K1 eingeführt. Die Vordestillationskolonne K1 ist in einem mittleren Abschnitt durch eine im Wesentlichen in Längsrichtung der Vordestillationskolonnne K1 ausgerichtete Trennwand 2 in einen Zulaufbereich 3 und einen Seitanabzugsbereich 4 unterteilt. Der Zulaufbereich 3 und der Seitenabzugsbereich 4 erstrecken sich in vertikaler Richtung jeweils vom oberen bis zum unteren Ende der Trennwand 2. Aus dem Seitanabzugsbereich 4 zieht man einen gasförmigen C₄-Schnitt 7 ab. Den Brüden der Vordestillationskolonne K1 führt man durch den Kopfkondensator 5. Das darin gebildete Kondensat führt man in die Vordestillationskolonne K1 zurück. Der nicht kondensierte Teil der Brüden bildet den Kopfstrom 6, der als eine Leichtsiederfraktion aus der Vordestillationskolonne K1 abgezogen wird. Aus der Vordestillationskolonne K1 zieht man außerdem eine erste Hochsiederfraktion als Sumpfstrom 9 ab. Der Sumpf der Vordestillationskolonne K1 wird über Verdampfer 8 beheizt.

Den gasförmigen C₄-Schnitt 7 führt man in einen unteren Abschnitt einer Extraktionskolonne K2 ein und bringt ihn darin mit einem ausgegastem selektiven Lösungsmittel 10 in Kontakt, das man in einen oberen Abschnitt der Extraktionskolonne K2 einspeist. Durch Kondensation des Brüdens der Extraktionskolonne K2 in Kondensator 11 erhält man eine Butane und Butene enthaltende Kopffraktion 12 (sogenanntes Raffinat I) sowie einen nicht kondensierten Anteil 13 des Brüdens. Der Strom 13 dient als das aus sicherheitstechnischen Gründen notwendige Verdünnungsgas für die Acetylene 32. Gleichzeitig ist der Strom 13 Auslassstrom für die Extraktionskolonne K2, um den Gehalt an molekularem Sauerstoff im Gasraum der Extraktionskolonne K2 zu kontrollieren und unter der Nachweisgrenze zu halten. Man erhält außerdem eine Sumpffraktion 14, die im Wesentlichen aus selektivem Lösungsmittel besteht, in dem 1,3-Butadien sowie Methylacetylen, Vinylacetylen, Ethylacetylen und C₅₊-Kohlenwasserstoffe gelöst sind.

Die Sumpffraktion 14 führt man in einen oberen Abschnitt 15 einer Extraktions- und Vorausgaserkolonne K3. Ein wesentlicher Teil des Roh-1,3-Butadiens wird in dem unteren Abschnitt 18 der Extraktions- und Vorausgaserkolonne K3 desorbiert. Das Roh-1,3-Butadien bringt man in einer Nachwaschzone mit ausgegastem selektivem Lösungsmittel 23 in Kontakt, um C₄-Acetylene abzutrennen. Die Nachwaschzone ist ein durch eine im Wesentlichen in Längsrichtung der Kolonne verlaufende Trennwand abgetrennter oberer Abschnitt 16 der Extraktions- und Vorausgaserkolonne K3. Man kondensiert das am Kopf der Extraktions- und Vorausgaserkolonne K3 aus der direkt oberhalb der Nachwaschzone befindlichen Waschsektion ausgeleitete Roh-1,3-Butadien 19 in Kondensator 20 teilweise. Den kondensierten Teil 21 des Roh-1,3-Butadiens führt man als Rücklauf in die Waschsektion über die der Rücklauf in die Nachwaschzone strömt. Den anderen Teil 22 des Roh-1,3-Butadiens führt man gasförmig der Reindestillationskolonne K5 zu. Der Sumpf der Extraktions- und Vorausgaserkolonne K3 wird über den Verdampfer 25 beheizt.

Ein am Kopf der Extraktions- und Vorausgaserkolonne K3 aus Abschnitt 15 ausgeleitetes, Butane und Butene enthaltendes Gas 17 führt man in einen unteren Abschnitt der Extraktionskolonne K2 zurück. Thermodynamisch entsprechen der Abschnitt 15 der Extraktions- und Vorausgaserkolonne K3 und die Extraktionskolonne K2 zusammen einer einzigen Extraktionskololonne, welche aus Gründen der Kolonnenhöhe vertikal zweigeteilt wurde.

Vorentgastes Lösungsmittel aus dem Sumpf der Extraktions- und Vorausgaserkolonne K3 wird als Strom 26 weiter in eine Ausgaserkolonne K4 geleitet. In der Ausgaserkolonne K4 desorbiert weiteres Roh-1,3-Butadien, das man über Leitung 27, den Direktkühler K6, den Kompressor 31 und Leitung 24 in den unteren Abschnitt der Extraktions- und Vorausgaserkolonne K3 führt. Über die Leitungen 29 und 30 wird Kühlmedium zu- und abgeführt. Als Kühlmedium dient Eigenkondensat, das im Wesentlichen aus Komponenten des selektiven Lösungsmittel, z.B. Wasser und NMP, besteht. Der Sumpf der Ausgaserkolonne K4 wird über Verdampfer 37 beheizt.

Ein C₄-Acetylene enthaltendes Gas 28 zieht man als Seitenabzug aus der Ausgaserkolonne K4 ab. Das C₄-Acetylene enthaltendes Gas 28 wird im Acetylenwäscher K7 mit Wasser gewaschen, das über Leitung 36 herangeführt wird. Der am Kopf des Acetylenwäschers K7 erhaltene Strom 32 wird mit dem Auslassstrom 13 der Extraktionskolonne K2 verdünnt. Kondensierbare Bestandteile werden im Kondensator 33 kondensiert und können teilweise als Rücklauf 35 auf den Acetylenwäscher K7 gegeben werden; der Rest ist im Wesentlichen Prozessabwasser. Nicht kondensierte Bestandteile werden als Strom 34 (verdünnter Acetylene-Strom) ausgeschleust.

In der Reindestillationskolonne K5 trennt man vom Roh-1,3-Butadien 22 eine zweite Hochsiederfraktion 43 ab. Man führt den Brüden in Kondensator 39. Der nicht kondensierte Teil der Brüden bildet den Auslassstrom 38. Das Rein-1,3-Butadien fällt im Kondensator 39 als Kondensat an und wird als Strom 40 entnommen; ein Teilstrom wird als Rücklauf auf die Reindestillationskolonne K5 gegeben. Der Sumpf der Reindestillationskolonne K5 wird über Verdampfer 42 beheizt.

Die zweite Hochsiederfraktion 43 führt man in einen unteren Abschnitt der Vordestillationskolonne K1 zurück. Den Entlüftungsstrom 38 führt man über das Gebläse 41 den Brüden der Vordestillationskolonne K1 zu.

### Beispiel 1

Das erfindungsgemäße Verfahren wurde auf Grundlage einer Anlage gemäß Figur 1 simuliert. Für die Simulationsrechnung wurde die BASF-eigene Software Chemasim verwendet; bei Verwendung kommerziell erhältlicher Software wie Aspen Plus (Hersteller: AspenTech, Burlington/Massachusetts, USA) oder PRO II (Fullerton, USA) würde man vergleichbare Ergebnisse erhalten. Der Parametersatz beruhte auf umfangreichen Gleichgewichtsmessungen, Untersuchungen an Laborkolonnen und Betriebsdaten verschiedener Anlagen. Die Zielspezifikation für das Rein-1,3-Butadien war: Mindestens 99,5% 1,3-Butadien, maximal 20 ppm 1,2-Butadien, maximal 20 ppm Acetylene.

Ausgegangen wurde von einem Roh-C₄-Schnitt enthaltend 1300 ppm C₃-Kohlenwasserstoffe, 2,0 % n-Butan, 0,6 % iso-Butan, 19,0 % n-Buten, 28,3 % iso-Buten, 5,5 % trans-2-Buten, 4,4 % cis-2-Buten, 39,0 % 1,3-Butadien, 0,2 % 1,2-Butadien, 1200 ppm 1-Butin, 4500 ppm Vinylacetylen und je 3000 ppm C₅-Kohlenwasserstoffe.

In Tabelle 1 sind die Massenströme und Zusammensetzungen relevanter Ströme zusammengefasst. Die Bezeichnungen der Ströme in der Tabelle beziehen sich auf die Bezugszeichen der Figur 1.

**Tabelle 1**

| **Beispiel 1** | Strom | | | |
|---|---|---|---|---|
| | 1 | 7 | 9 | 43 |
| Massenstrom [kg/h] | 32000 | 31935 | 200 | 37 |
| 1,2-Butadien [Gew.-%] | 0,15 | 0,07 | 12,13 | 40,00 |
| Acetylene [Gew.-%] | 0,57 | 0,57 | 0,38 | 0,20 |
| C₅₊-Komponenten [Gew.-%] | 0,30 | 0,02 | 45,00 | 8,97 |
| 1,3-Butadien [Gew.-%] | | | 3,63 | 35,94 |

Über den einzigen Hochsiederauslassstrom 9 ergibt sich ein Verlust an 1,3-Butadien von etwa 7 kg/h. Der Gehalt der C₅-Komponenten als die am schwierigsten abzutrennenden Hochsieder wird in der Vordestillationskolonne um etwa 94% reduziert.

### Vergleichsbeispiel 2

Es wurde ein Verfahren des Stands der Technik simuliert. Die Zusammensetzung des Roh-C₄-Schnitts und die Zielspezifikation für das Rein 1,3-Butadien stimmten mit Beispiel 1 überein. Die zugrunde gelegte Anlage wies an Stelle der mit der Trennwand 2 versehenen Vordestillationskolonne K1 eine vorgeschaltete Destillationskolonne gemäß WO 2013/083536 A1 auf, der der flüssiger Roh-C₄-Schnitt zugeführt wurde. Eine Rückführung der Hochsiederfraktion aus der Reindestillationskolonne in die Vordestillationskolonne ist nicht vorgesehen.

In Tabelle 2 sind die Massenströme und Zusammensetzungen relevanter Ströme zusammengefasst.

**Tabelle 2**

| **Vergleichsbeispiel 2** | Strom | | | |
|---|---|---|---|---|
| | Roh-C₄ fl.¹ | Roh-C4 ger.² | Sumpf Dest.³ | Sumpf Rein⁴ |
| Massenstrom [kg/h] | 32000 | 31743 | 110 | 69 |
| 1,2-Butadien [Gew.-%] | 0,15 | 0,14 | 40,00 | 40,00 |
| Acetylene [Gew.-%] | 0,57 | 0,57 | 0,21 | 0,18 |
| C₅₊-Kom ponenten [Gew.-%] | 0,30 | 0,13 | 8,38 | 30,18 |
| 1,3-Butadien [Gew.-%] | | | 29,88 | 14,88 |

| | | | | |
|---|---|---|---|---|
| 1 flüssiger Roh-C₄-Schnitt (Bezugszeichen 1 in Fig. 1 der WO 2013/083536 A1) 2 dampfförmiger gereinigter Roh-C₄-Schnitt (Bezugszeichen 4 in Fig. 1 der WO 2013/083536 A1) 3 an der vorgeschalteten Destillationskolonne anfallender Sumpfstrom (Bezugszeichen 3 in Fig. 1 der WO 2013/083536 A1) 4 bei der Reindestillation anfallender Sumpfstrom | | | | |

Bei diesem Verfahren fallen zwei C₅₊-haltige Hochsiederströme an, die verworfen werden: Der Sumpfstrom der vorgeschalteten Destillationskolonne und der bei der Reindestillation anfallende Sumpfstrom. Es ergibt sich ein Verlust an 1,3-Butadien von etwa 43 kg/h. In der vorgeschalteten Destillationskolonne des Vergleichsbeispiels 2 wurde nur ein geringerer Anteil der C₅₊-Komponenten abgetrennt (55 %). Über den dampfförmigen gereinigten Roh-C₄-Schnitt wurden die C₅₊-Komponenten zu einem weitaus höheren Anteil in die Extraktionskolonne verschleppt.

### Beispiel 3: Vergleich der Auslassströme zur Sauerstoffausschleusung

Tabelle 3 vergleicht die Massenströme der üblichen Auslassströme zur Sauerstoffausschleusung mit den erfindungsgemäß vorgeschlagenen internen Strömen zur Sauerstoffausschleusung. Die Tabelle illustriert, dass durch das Ersetzen von Auslassströmen durch interne Ströme deren Massenstrom ohne Verlust an Wertprodukt vervielfacht werden kann. Über die gemäß Stand der Technik üblichen Auslassströme werden typischerweise insgesamt pro Stunde etwa 30 bis 60 Kg 1,3-Butadien enthaltender Gase ausgeleitet. Die Ströme 13, 22 und 38 ersetzen in der Anlage der Figur 1 je einen Auslassstrom. Dennoch ist der über diese Ströme auftretende Verlust an Wertprodukt geringer, da die Ströme in das Verfahren zurückgeführt werden. Durch die Vervielfachung der Massenströme von 10 bis 20 auf 360 kg/h, 18376 kg/h bzw. 360 kg/h wird aus den Kolonnen mehr Sauerstoff abgeführt. Dadurch lässt sich der Sauerstoffgehalt unter die Nachweisgrenze üblicher Detektoren drücken und folglich auch die unerwünschte Polymerisationsneigung des 1,3-Butadiens verringern.

**Tabelle 3**

| Stand der Technik | | Erfindungsgemäßes Verfahren (Figur 1) | |
|---|---|---|---|
| Auslassstrom am | | Strom | |
| Kopf der Extraktionskolonne | 10-20 kg/h | 13 | 360 kg/h |
| Kopf der Extraktions- und Vorausgaserkolonne | 10-20 kg/h | 22 | 18376 kg/h |
| Kopf der Reindestillationskolonne | 10-20 kg/h | 38 | 200 kg/h |
| Summe | 30-60 kg/h | Summe | 18936 kg/h |
| Verlust | 30-60 kg/h | Verlust | 0 |

## Patentansprüche

1. Verfahren zur Gewinnung von Rein-1,3-Butadien aus einem Roh-C₄-Schnitt durch Extraktivdestillation mit einem selektiven Lösungsmittel, wobei man
a) einen flüssigen Roh-C₄-Schnitt in den Zulaufbereich einer Vordestillationskolonne einführt, welche in einem mittleren Abschnitt durch eine im Wesentlichen in Längsrichtung der Vordestillationskolonnne ausgerichtete Trennwand in einen Zulaufbereich und einen Seitenabzugsbereich unterteilt ist, eine C₃-Kohlenwasserstoffe enthaltende erste Leichtsiederfraktion als Kopfstrom, einen gasförmigen C₄-Schnitt als Seitenstrom aus dem Seitenabzugsbereich und eine erste Hochsiederfraktion als Sumpfstrom abzieht,
b) den gasförmigen C₄-Schnitt in wenigstens einer Extraktionskolonne mit einem selektiven Lösungsmittel in Kontakt bringt, wobei man eine Butane und Butene enthaltende Kopffraktion und eine 1,3-Butadien und selektives Lösungsmittel enthaltende Sumpffraktion erhält,
c) aus der Sumpffraktion in wenigstens einer Ausgaserkolonne Roh-1,3-Butadien desorbiert, wobei man ein ausgegastes selektives Lösungsmittel erhält und das ausgegaste selektive Lösungsmittel in die Extraktionskolonne zurückführt, und
d) wenigstens einen Teil des Roh-1,3-Butadiens einer Reindestillationskolonne zuführt und eine zweite Hochsiederfraktion abtrennt und einen gasförmigen Auslassstrom abzieht und die zweite Hochsiederfraktion in einen unteren Abschnitt der Vordestillationskolonne zurückführt.

2. Verfahren nach Anspruch 1, wobei man den Auslassstrom aus der Reindestillationskolonne zusammen mit dem Brüden der Vordestillationskolonne durch den Kopfkondensator der Vordestillationskolonne führt.

3. Verfahren nach einem der vorhergehenden Ansprüche, wobei man das Roh-1,3-Butadien in einer Nachwaschzone mit ausgegastem selektiven Lösungsmittel in Kontakt bringt.

4. Verfahren nach Anspruch 2 oder 3, wobei man das desorbierte Roh-1,3-Butadien teilweise kondensiert, den kondensierten Teil des Roh-1,3-Butadiens als Rücklauf in die Ausgaserkolonne und/oder in die Nachwaschzone führt und den anderen Teil des Roh-1,3-Butadiens gasförmig der Reindestillationskolonne zuführt.

5. Verfahren nach einem der vorhergehenden Ansprüche, wobei man den gasförmigen C₄-Schnitt in einer Extraktionskolonne und in einem oberen Abschnitt einer Extraktions- und Vorausgaserkolonne mit dem selektiven Lösungsmittel in Kontakt bringt und das Roh-1,3-Butadien in einem unteren Abschnitt der Extraktions- und Vorausgaserkolonne und einer Ausgaserkolonne von der Sumpffraktion desorbiert.

6. Verfahren nach Anspruch 5, wobei die Nachwaschzone ein durch eine im Wesentlichen in Längsrichtung der Kolonne verlaufende Trennwand abgetrennter oberer Abschnitt der Extraktions- und Vorausgaserkolonne ist.

7. Verfahren nach einem der Ansprüche 2 bis 6, wobei man aus der Sumpffraktion außerdem ein C₄-Acetylene enthaltendes Gas desorbiert.

8. Verfahren nach Anspruch 7, wobei man das C₄-Acetylene enthaltende Gas als Seitenabzug aus der Ausgaserkolonne abzieht.

9. Verfahren nach Anspruch 7 oder 8, wobei man das C₄-Acetylene enthaltende Gas mit nicht kondensierten Bestandteilen des Brüdens der Extraktionskolonne verdünnt.

10. Verfahren nach einem der vorhergehenden Ansprüche, wobei der Roh-C₄-Schnitt
- 15 bis 85 Gew.-% 1,3-Butadien,
- 4 bis 13 Gew.-% Butane,
- 24 bis 64 Gew.-%, Butene,
- 0,2 bis 0,5 Gew.-% C₄-Acetylene,
- 0,01 bis 2,0 Gew.-% C₃-Kohlenwasserstoffe und
- 0,01 bis 0,5 Gew.-% C₅₊-Kohlenwasserstoffe
enthält.

11. Verfahren nach einem der vorhergehenden Ansprüche, wobei das selektive Lösungsmittel wenigstens 80 Gew.-% N-Methylpyrrolidon enthält.

## Claims

1. A process for isolating pure 1,3-butadiene from a crude C₄ fraction by extractive distillation using a selective solvent, wherein
a) a liquid crude C₄ fraction is introduced into the inflow region of a predistillation column, which is divided in a middle section by a dividing wall aligned essentially in the longitudinal direction of the predistillation column into an inflow region and a side offtake region, a first low boiler fraction comprising C₃-hydrocarbons is taken off as overhead stream, a gaseous C₄ fraction is taken off as side stream from the side offtake region and a first high boiler fraction is taken off as bottom stream,
b) the gaseous C₄ fraction is brought into contact with a selective solvent in at least one extraction column, giving an overhead fraction comprising butanes and butenes and a bottom fraction comprising 1,3-butadiene and selective solvent,
c) crude 1,3-butadiene is desorbed from the bottom fraction in at least one stripping column, with a stripped selective solvent being obtained and the stripped selective solvent being recirculated to the extraction column, and
d) at least part of the crude 1-3-butadiene is fed to a pure distillation column and a second high boiler fraction is separated off and a gaseous purge stream is taken off and the second high boiler fraction is recirculated to a lower section of the predistillation column.

2. The process according to claim 1, wherein the purge stream from the pure distillation column is conveyed together with the vapor from the predistillation column through the overhead condenser of the predistillation column.

3. The process according to any of the preceding claims, wherein the crude 1,3-butadiene is brought into contact with stripped selective solvent in an after-scrubbing zone.

4. The process according to claim 2 or 3, wherein the desorbed crude 1,3-butadiene is partially condensed, the condensed part of the crude 1,3-butadiene is conveyed as runback into the stripping column and/or into the after-scrubbing zone and the other part of the crude 1,3-butadiene is fed in gaseous form to the pure distillation column.

5. The process according to any of the preceding claims, wherein the gaseous C₄ fraction is brought into contact with the selective solvent in an extraction column and in an upper section of an extraction and prestripping column and the crude 1,3-butadiene is desorbed from the bottom fraction in a lower section of the extraction and prestripping column and a stripping column.

6. The process according to claim 5, wherein the after-scrubbing zone is an upper section of the extraction and prestripping column separated off by a dividing wall running essentially in the longitudinal direction of the column.

7. The process according to any of claims 2 to 6, wherein a gas comprising C₄-acetylenes is also desorbed from the bottom fraction.

8. The process according to claim 7, wherein the gas comprising C₄-acetylenes is taken off as side offtake stream from the stripping column.

9. The process according to claim 7 or 8, wherein the gas comprising C₄-acetylenes is diluted with uncondensed constituents of the vapor from the extraction column.

10. The process according to any of the preceding claims, wherein the crude C₄ fraction comprises
- from 15 to 85% by weight of 1,3-butadiene,
- from 4 to 13% by weight of butanes,
- from 24 to 64% by weight of butenes,
- from 0.2 to 0.5% by weight of C₄-acetylenes,
- from 0.01 to 2.0% by weight of C₃-hydrocarbons and
- from 0.01 to 0.5% by weight of C₅₊-hydrocarbons.

11. The process according to any of the preceding claims, wherein the selective solvent comprises at least 80% by weight of N-methylpyrrolidone.

## Revendications

1. Procédé pour l'obtention de 1,3-butadiène pur à partir d'une fraction brute en C₄ par distillation extractive avec un solvant sélectif, dans lequel
a) on introduit une fraction brute en C₄ dans la partie d'admission d'une colonne de pré-distillation qui est, dans une portion centrale, divisée en une partie d'admission et une partie de soutirage latéral par l'intermédiaire d'une paroi de séparation orientée essentiellement dans la direction longitudinale de la colonne de pré-distillation, on soutire en tant que flux de tête une première fraction volatile contenant des hydrocarbures en C₃, on soutire en tant que flux latéral une fraction gazeuse en C₄ de la partie de soutirage latéral et on soutire une première fraction non volatile en tant que flux de fond,
b) on met en contact la fraction gazeuse en C₄ avec un solvant sélectif dans au moins une colonne d'extraction, dans lequel on obtient une fraction de tête contenant des butanes et des butènes et une fraction de fond contenant du 1,3-butadiène et du solvant sélectif,
c) on désorbe le 1,3-butadiène brut de la fraction de fond dans au moins une colonne dégazage, dans lequel on obtient un solvant sélectif dégazé et on renvoie le solvant sélectif dégazé dans la colonne d'extraction, et
d) on alimente au moins une partie du 1,3-butadiène brut à une colonne de distillation pure et on sépare une deuxième fraction non volatile et on soutire un flux d'échappement gazeux et on renvoie la deuxième fraction non volatile dans une portion inférieure de la colonne de pré-distillation.

2. Procédé selon la revendication 1, dans lequel on fait passer le flux d'échappement de la colonne de distillation pure conjointement avec des vapeurs de la colonne de pré-distillation par le condensateur de tête de la colonne de pré-distillation.

3. Procédé selon l'une quelconque des revendications précédentes, dans lequel on met en contact le 1,3-butadiène brut dans une zone de post-lavage avec du solvant sélectif dégazé.

4. Procédé selon la revendication 2 ou 3, dans lequel on condense partiellement le 1,3-butadiène brut désorbé, on alimente la partie condensée du 1,3-butadiène brut en tant que retour dans la colonne de dégazage et/ou dans la zone de post-lavage et on alimente l'autre partie du 1,3-butadiène brut gazeux à la colonne de distillation pure.

5. Procédé selon l'une quelconque des revendications précédentes, dans lequel on met en contact la fraction gazeuse en C₄ dans une colonne d'extraction et dans une portion supérieure d'une colonne d'extraction et de pré-dégazage avec le solvant sélectif et on désorbe de la fraction de fond le 1,3-butadiène brut dans une portion inférieure de la colonne d'extraction et de pré-dégazage et d'une colonne de dégazage.

6. Procédé selon la revendication 5, la zone de post-lavage étant une portion supérieure de la colonne d'extraction et de pré-dégazage divisée par une paroi de séparation s'étendant essentiellement dans la direction longitudinale de la colonne.

7. Procédé selon l'une quelconque des revendications 2 à 6, dans lequel on désorbe en outre de la fraction de fond un gaz contenant des acétylènes en C₄.

8. Procédé selon la revendication 7, dans lequel on soutire le gaz contenant des acétylènes en C₄ comme soutirage latéral de la colonne de dégazage.

9. Procédé selon la revendication 7 ou 8, dans lequel on dilue le gaz contenant des acétylènes en C₄ avec des ingrédients non condensés des vapeurs de la colonne d'extraction.

10. Procédé selon l'une quelconque des revendications précédentes, la fraction brute en C₄ contenant
- 15 à 85 % en poids de 1,3-butadiène,
- 4 à 13 % en poids de butanes,
- 24 à 64 % en poids, de butènes,
- 0,2 à 0,5 % en poids d'acétylènes en C₄,
- 0,01 à 2,0 % en poids d'hydrocarbures en C₃ et
- 0,01 à 0,5 % en poids d'hydrocarbures en C₅₊.

11. Procédé selon l'une quelconque des revendications précédentes, le solvant sélectif contenant au moins 80 % en poids de N-méthylpyrrolidone.
